# EUROPÄISCHE PATENTSCHRIFT

(11) **EP 1 110 544 B1**
(45) Veröffentlichungstag und Bekanntmachung des Hinweises auf die Patenterteilung: **13.02.2008**
(21) Anmeldenummer: 00125170.1
(22) Anmeldetag: 18.11.2000
(51) Int. Cl.: A61K 9/28

(54) **Verfahren zur Herstellung einer geschmacksmaskierten oralen Darreichungsform& x9;**
Method of producing a taste-masked oral preparation
Méthode pour fabriquer une préparation orale masquant le goût

(30) Priorität: 20.12.1999 DE 19961897
(43) Veröffentlichungstag der Anmeldung: 27.06.2001
(73) Patentinhaber: BASF Aktiengesellschaft, 67056 Ludwigshafen (DE)
(72) Erfinder: Kolter, Karl, Dr., 67117 Limburgerhof (DE); Scheiffele, Silke, 67269 Grünstadt (DE); Einig, Heinz, Dr., 67433 Neustadt (DE); Bodmeier, Roland, Prof. Dr., 14163 Berlin (DE)

(56) Entgegenhaltungen:
- EP-A- 0 211 991
- EP-A- 0 458 751
- EP-A- 0 868 912
- WO-A-00/56266
- DE-A- 3 943 242
- US-A- 4 800 087
- US-A- 4 871 546
- US-A- 5 780 021

## Beschreibung

Die vorliegende Erfindung betrifft ein Verfahren zur Herstellung von geschmacksmaskierten oralen Darreichungsformen durch Verpressen von mit einem Filmüberzug versehenen Partikeln mit üblichen Tablettierhilfsstoffen zu Tabletten, unter Verwendung eines Filmüberzuges, enthaltend Polyvinylacetat, hydrophile Zusätze aus der Gruppe der filmbildenden wasserlöslichen Polymere, der wasserunlöslichen, quellenden Polymere und/oder der Gruppe der feinteiligen Puderungsmittel als geschmacksmaskierendes Coating.

Orale Darreichungsformen werden vom Patienten in Form von Lösungen, Emulsionen, Suspensionen, Kapseln und Tabletten eingenommen, wobei den festen Formen aufgrund ihrer guten Dosierbarkeit, Verpackung, Transportfähigkeit, Haltbarkeit und zuletzt der einfachen Einnahme die größte Bedeutung zukommt. Viele Arzneistoffe schmecken bitter, von daher muß entweder der Kontakt des Arzneistoffes mit der Mund- und Rachenschleimhaut verhindert werden oder der bittere Geschmack überdeckt werden.

Bei festen Darreichungsformen, die unzerkaut geschluckt werden, kann die gesamte Darreichungsform geschützt werden z.B. durch die Abfüllung in Kapseln, durch Auftrag einer Coatingschicht auf die Tablette bzw. die Herstellung von Tabletten mit hoher Bruchfestigkeit und langsamem Zerfall. Diese Art der Geschmacksmaskierung ist aber nicht anwendbar bei Darreichungsformen, die vor oder bei der Applikation zerstört werden, z.B. durch Zerkauen oder Auflösen/Dispergieren in Wasser. Kinder, ältere Menschen und viele andere Patienten haben Probleme, Tabletten und Kapseln unzerstört einzunehmen.

Wirkstoffe, bei denen die Dosierung eine kleine, leicht schluckbare Tablette nicht zuläßt, sollten als flüssige Darreichungsform oder als kaubare Tablette angeboten werden. Wünschenswert ist die letztere Variante, da flüssige Darreichungsformen die oben beschriebenen Vorteile nicht aufweisen.

Ein bekanntes Problem bei Kautabletten ist das Auftreten des schlechten Geschmacks des Wirkstoffs während des Kauvorgangs. Der Geschmack des Wirkstoffs kann durch Zusatz von Aromastoffen und Süßungsmitteln verbessert werden, so dass während des Kauvorgangs der bittere Geschmack überdeckt wird. Um eine ausreichende Maskierung des Wirkstoffs mit Aromastoffen und Süßungs mitteln während des Kauvorgangs zu erhalten, wird oft ein hoher Anteil jener Stoffe benötigt, so dass auf diese Art lediglich die Herstellung von Tabletten mit geringer Wirkstoffkonzentration möglich ist.

Anwendung findet diese Methode der Geschmacksmaskierung bei Darreichungsformen für Kinder, bei denen die Wirkstoffkonzentration niedrig ist und somit der Anteil an Aromastoffen und Süßungsmitteln, die für eine Maskierung notwendig sind, die Tablette nicht ungewöhnlich groß werden läßt. Bei vielen Wirkstoffen z.B. Ibuprofen ist aufgrund des dominierenden Geschmacks mit dieser Methode keine ausreichende Maskierung möglich.

Eine weitere Möglichkeit, den Geschmack des Wirkstoffs während des Kauvorganges zu maskieren ist das Coating. Wirktoffhaltige Formlinge werden mit einem geschmacksmaskierenden Coating überzogen und anschließend zu Tabletten verpresst. Während des Kauvorgangs der Tablette wird durch die Umhüllung die Freisetzung des Wirkstoffs verhindert, so dass kein bitterer Geschmack entsteht. Nach dem Schlucken der zerkauten Tablette ist eine schnelle Freisetzung des Wirkstoffs erwünscht, um eine Verzögerung des Wirkungseintritts zu vermeiden.

Ein Überzug zur Herstellung von Tabletten mit verzögerter Wirkstofffreisetzung wird in US-Patent 4415547 beschrieben. Pellets werden mit einer organischen Sprühlösung bestehend aus einem hydrophilen Polymer (PVP), einem hydrophoben Polymer (Ethylcellulose) und weiteren üblichen Coatingbestandteilen überzogen und anschließend unter Einarbeitung weiterer Hilfsstoffe zu Tabletten verpresst.

In der EP-A 868 912 ist die Verwendung eines Überzugsmaterials aus Polyvinylacetat, einem N-Vinylpyrrolidonhaltigen Polymeren und weiteren Hilfsstoffen zur Herstellung von Filmüberzügen auf pharmazeutischen oder agrochemischen Darreichungsformen beschrieben, die eine kontrollierbare Auflösung der in den Darreichungsformen enthaltenen Wirkstoffen ermöglichen. Eine Verpressung von mit entsprechenden Überzeugungsmaterialien versehenen Partikeln zu Tabletten ist nicht beschrieben.

Das Patent EP 317274 beschreibt einen geschmacksmaskierenden Überzug basierend auf Celluloseacetat oder Celluloseacetatbutyrat und Polyvinylpyrrolidon. Die Polymere werden in organischen Lösungsmitteln gelöst, wobei der Feststoffgehalt der Sprühlösung zwischen 8 und 10% liegt. Die für die Geschmacksmaskierung notwendige Auftragsmenge wird mit 12-15 Gew.% angegeben.

Patent EP 523847 erläutert eine Polymermischung bestehend aus Methylaminoethylmethacrylat und neutralem Methacrylsäueester und einem Celluloseester sowie PVP. Eine 10%ige, organische Lösung wird auf die zu überziehende pharmazeutisch wirksame Zusammensetzung aufgesprüht.

Eine Kombination von hydrophobem Polymer (EA:MMA) und wasserunlöslichem, aber quellendem Polymer wird zur Geschmacksmaskierung in Patent EP 570606 eingesetzt. Der Zusatz des wasserunlöslichen Polymers hat außerdem die Aufgabe, die Klebrigkeit des hydrophoben Polymers zu reduzieren, so dass eine Adhesion der gecoateten Partikel vermieden wird.

Die hier angeführten Überzugsmaterialien haben den Nachteil, daß sie eine nur sehr geringe Elastizität aufweisen. Bei starken mechanischen Belastungen der gecoateten Partikel kann es während des Press- oder Kauvorgangs der Tablette im Mund zu Rißbildungen kommen, durch die während der Mundpassage Wirkstoff diffundiert und einen bitteren Geschmack entstehen läßt.

Neben der schlechten Geschmacksmaskierung führen viele dieser Filmüberzüge zu Verarbeitungsproblemen, wie z.B. Zusammenkleben der Pellets oder Kristalle.

Außerdem werden die meisten der schon für die Geschmacksmaskierung beschriebenen Polymere in organische Lösungsmittel eingearbeitet. Nachteile organischer Sprühlösungen sind bekannterweise die hohen Kosten, die Risiken für Mensch und Umwelt und nicht zuletzt der Verbleib einer Restmenge in der Arzneiform.

Der vorliegenden Erfindung lag daher die Aufgabe zugrunde, ein Polymerisat zur Geschmacksmaskierung von oralen, insbesondere pharmazeutischen Darreichungsformen bereitzustellen, das die oben angeführten Nachteile nicht aufweist.

Die Aufgabe wurde erfindungsgemäß gelöst, durch ein Verfahren zur Herstellung einer geschmackmaskierten oralen Darreichungsform durch Verpressung von mit einem Filmüberzug versehenen wirkstoffhaltigen Partikeln mit üblichen Tablettierhilfsstoffen zu Tabletten, dadurch gekennzeichnet, dass die wirkstoffhaltigen Partikel mit einem geschmacksmaskierenden Filmüberzug aus
a) Polyvinylacetat
b) einem hydrophilen Zusatz ausgewählt aus der Gruppe der filmbildenden wasserlöslichen Polymeren, und/oder der Gruppe der wasserunlöslichen, aber quellenden Polymeren und/oder der Gruppe der sehr feinteiligen Puderungsmittel, wobei die filmbildenden wasserlöslichen Polymere ausgewählt sind aus der Gruppe der Poly(vinyllactame), Vinylpyrrolidon-Vinylacetat-Copolymere, Polyvinylalkohole oder Cellulosederivate, als wasserunlösliche, aber stark quellende Polymere quervernetzte Poly(vinyllactame), Cellulose bzw. Cellulosederivate oder Stärkederivate und als feinteilige Puderungsmittel hochdisperse KieSelsäuren, feinteilige Stärken, feinteilige Cellulosen oder feinteilige Salze der Phosphorsäure,
mit einem Gewichtsverhältnis von Polyvinylacetat zu hydrophilem Zusatz von 1:0,1 bis 1:0,75 überzogen werden und in Gegenwart von üblichen Tablettierhilfsmitteln zu Tabletten verpresst werden.

Coatings basierend auf Polyvinylacetat und hydrophilen Zusätzen zur Geschmacksmaskierung von arzneistoffhaltigen Formlingen, können direkt appliziert oder anschließend mit weiteren üblichen Tablettierhilfsstoffen zu Tabletten verpreßt werden. Die Polyvinylacetatdispersionen werden ohne organische Lösungsmittel hergestellt und weisen somit den Vorteil des hohen Feststoffgehalts einer wässrigen Dispersion auf, was zu einer kürzeren Prozesszeit und somit zu erheblichen Einsparungen von Energie und Zeit führt.

Gegenüber Lösungen besitzen sie außerdem den Vorteil des hohen Feststoffanteils der Sprühsuspension, was zu einer kürzeren Prozesszeit und somit zu einer Einsparung von Energie und Zeit führt.

Aufgrund der großen Plastizität und somit hoher Stabilität gegenüber mechanischen Eigenschaften ist das Coatingmaterial ideal geeignet, um arzneistoffhaltige Formlinge geschmacksmaskierend zu coaten und anschließend zu tablettieren ohne, dass es zu Beschädigungen des Überzugs kommt.

Das beschriebene Coatingmaterial weist sowohl während des Sprühprozesses als auch während der weiteren Verarbeitung keine Klebrigkeit auf. Die verpressten, gecoateten Partikel zerfallen bei dem Kauvorgang bzw. bei Zufuhr von Flüssigkeit wieder in die Primärpartikel.

Die weitaus bessere Elastizität des Coatings gegenüber Ethylcellulose oder anderen vorher beschriebenen Produkten ist bei starken mechanischen Belastungen der gecoateten Partikel von Vorteil. Durch die hohe Flexibilität des Überzuges kommt es weder während des Pressvorgangs noch bei dem Kauvorgang der Tablette zu Rißbildungen, durch die während der Mundpassage Wirkstoff diffundiert und einen bitteren Geschmack entstehen läßt.

Obwohl Polyvinylacetat wasserunlöslich ist, kann es leicht quellen und Wasser permeieren lassen. Dies ist ein entscheidender Vorteil gegenüber anderen lipophilen Coatingpolymeren, die kaum Wasser permeieren lassen und so die Wirkstofffreisetzung stark verzögern. Die erfindungsgemäßen Coatingzubereitungen ermöglichen eine starke Geschmacksmaskierung bei dennoch sehr schneller Wirkstofffreisetzung.

Das in der vorliegenden Erfindung beschriebene Coatingmaterial weist sowohl während des Sprühprozesses als auch während der weiteren Verarbeitung keine Klebrigkeit auf. Dadurch ist ein reproduzierbares Coating der Partikel ohne Zwillings- bzw. Mehrlingsbildung während des Sprühprozesses möglich. Polyvinylacetat zeigt ein ausgezeichnetes Spreitungsverhalten und haftet sehr gut auf dem Kern. Die Weiterverarbeitung der gecoateten Formlinge ist möglich, ohne dass es durch Adhesion zu Dosierungsschwankungen bzw. Problemen bei der Gleichförmigkeit des Gehaltes kommt. Trotz anschließender Verpressung der gecoateten Partikel, zerfallen diese bei dem Kauvorgang bzw. bei Zufuhr von Flüssigkeit in die Primärpartikel und zeigen somit die Vorteile einer multiple unit dosage form auf.

Das eingesetzte Polyvinylacetat ist kompatibel mit löslichen und unlöslichen hydrophilen Polymeren, und bildet einen äußerst stabilen Sprühansatz.

Diese Sprühsuspension wird zum Überziehen wirkstoffhaltiger Formlinge eingesetzt, wobei die Geschmacksmaskierung und die Geschwindigkeit der Freisetzung durch das Verhältnis von Polyvinylacetatdispersion und hydrophilem Zusatzstoff eingestellt werden können.

Die gecoateten Partikel werden anschließend mit weiteren üblichen Tablettierhilfsstoffen verpresst. Aufgrund der hohen mechanischen Stabilität des Coatings kommt es weder durch das Tablettieren noch durch den Kauvorgang zu Beschädigungen, die die gewünschte Funktion beeinträchtigen. Durch den Kauvorgang bzw. die Zufuhr von Flüssigkeit werden die Primärpartikel wieder erhalten.

Da durch das intakte Coating eine optimale Geschmacksmaskierung erzielt wird, kann der Zusatz von Aromastoffen und Süßungsmitteln in der Kautablette oft reduziert werden.

Sobald die Tablette zerkaut und die gecoateten Partikel geschluckt werden, wird der Wirkstoff entweder durch Permeation durch das Coating oder durch Herauslösen des hydrophilen Anteils aus der Umhüllung freigesetzt.

Polyvinylacetat weist keine geladenen oder ionisierbaren Gruppen auf. Es ist unlöslich in Wasser und somit für die Herstellung von Retardformulierungen mit pH-unabhängiger Wirkstofffreisetzung geeignet.

Durch Zusatz an hydrophilen Stoffen wird überraschenderweise eine ausgezeichnete Geschmacksmaskierung bei gleichzeitig schneller Wirkstofffreisetzung erzielt.

Bei der Verwendung des Coatingmaterials zur Geschmacksmaskierung von Wirkstoffen bestimmt der hydrophile Anteil die Zeitdauer der geschmacksmaskierenden Wirkung in der Mundpassage. Der hydrophile Anteil bestimmt die Permeabilität des Films. Aus diesem Grund wird eine aus mit reinem Polyvinylacetat gecoateten Partikel hergestellte Kautablette eine hervorragende Geschmacksmaskierung erzielen, jedoch den Wirkstoff nach dem Schlucken nicht schnell genug freisetzen.

Durch hydrophile Zusätze kann die Freisetzung beschleunigt werden unter gleichzeitiger Beibehaltung der ausgezeichneten Geschmacksmaskierung.

Der Anteil des hydrophilen Zusatzes muss gewährleisten, daß eine gute Geschmacksmaskierung vorhanden ist während die Tablette im Mund zerkaut wird, des weiteren sollte nach dem Schluckvorgang eine schnelle Freisetzung des Wirkstoffes erfolgen. Das Verhältnis von Polyvinylacetat zu hydrophilem Zusatz liegt zwischen 1 : 0,1 bis 1 : 0,75 bevorzugt zwischen 1 : 0,2 bis 1 : 0,5.

Als hydrophile Zusatzstoffe werden in der Regel filmbildende wasserlösliche Polymere, wasserunlösliche aber quellende Polymere oder feinteilige Puderungsmittel eingesetzt, weiterhin können Zucker wie Dextrose oder Saccharose zugesetzt werden, Oligosaccharide bzw. Zuckerpolymere.

Als wasserlösliche filmbildende Polymere können Poly(vinyllactame), Vinylacetat-Vinylpyrrolidon-copolymere, Polyvinylalkohole oder Cellulosederivate verwendet werden. Beispiele sind Povidon, Copovidon, Polyvinylalkohol, Hydroxypropylcellulose, Hydroxypropylmethylcellulose, Hydroxyethylcellulose.

Die wasserunlöslichen aber quellenden Polymere umfassen quervernetzte Polyvinylpyrrolidone, quervernetzte Cellulose bzw. Cellulosederivate oder quervernetzte Stärke bzw. Stärkederivate. Beispiele hierfür sind Crospovidon, Croscarmellose und quervernetzte Natriumcarboxymethylstärke.

Als Puderungsmittel können hochdisperse Kieselsäuren, feinteilige Stärken oder Cellulosen oder feinteilige Salze der Phosphorsäure verwendet werde. Es ist häufig vorteilhaft, Stoffe aus den genannten Gruppen miteinander zu kombinieren.

Der Sprühzubereitung können weitere übliche Bestandeile zugesetzt werden. Dazu gehören Weichmacher um die Flexibilität des Überzuges einzustellen. Geeignete Weichmacher für Polyvinylacetat sind z.B. Propylenglykol, Triacetin, Triethylcitrat, Acetyltributylcitrat, Polyethylenglykole, Pyrrolidon. Weitere Bestandteile sind Antiklebemittel, wie z.B. Talkum oder Glycerolmonostearat, Farbstoffe, wie z.B. Eisenoxide oder Chinolingelb, Benetzungsmittel, wie z.B. Natriumlaurylsulfat oder Cremophor RH 40, und Entschäumer, wie z.B. Simethicon.

Als physiologisch verträgliche Säuren können z.B. verwendet werden: Sulfonsäure, Pivalinsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Napthalin-mono- und -disulfonsäure, Laurylschwefelsäure, Butandisulfonsäure.

Als Basen können beispielsweise Natrium- oder Kaliumhydroxid, Natrium- oder Kaliumcarbonat verwendet werden.

Der Zusatz von Säure im Filmüberzug kann die Löslichkeit von Wirktoffen und die Permeation durch den Filmüberzug herabsetzen und auf diese Weise zusätzlich zur Geschmacksmaskierung beitragen.

Die Herstellung des Coatings kann im Gegensatz zu den in anderen Patenten aufgeführten Formulierungen auch auf wässriger Basis erfolgen. Die Polyvinylacetatdispersion wird ohne organische Lösungsmittel hergestellt. Die hydrophilen Zusätze werden zuerst in Wasser gelöst bzw. suspendiert und anschließend in die Polyvinylacetatdispersion eingearbeitet. Die so hergestellte Sprühsuspension besitzt einen hohen Feststoffanteil, was zu einer kürzeren Prozesszeit und somit zu einer Einsparung von Energie und Zeit führt. Bei wasserempfindlichen Wirkstoffen ist Polyvinylacetat als organische Lösung einsetzbar, wobei Methanol oder Aceton als Lösungsmittel dienen. Die hydrophilen Zusätze können auch direkt der Polymerdispersion zugegeben werden.

Für das geschmacksmaskierende Coating können sowohl pulvrige Substanzen als auch Granulate, Pellets, Kristalle sowie Tabletten eingesetzt werden. Um eine optimale Geschmacksmaskierung und auch eine schnelle Freisetzung nach dem Schluckvorgang zu erzielen, können auch Wirkstoffpellets mit einem hohen Anteil an Sprengmittel hergestellt werden. Die verdichtete, runde Form sowie die glatte Oberfläche der Pellets ermöglichen einen gleichmäßigen, intakten Filmüberzug, so dass die Geschmacksmaskierung während des Kauvorgangs gewährleistet ist. Das gegebenenfalls in die Pellets eingearbeitete Sprengmittel sorgt anschließend für einen schnellen Zerfall der Partikel und somit für eine zügige Freisetzung des Wirkstoffs. Durch diese Kombination wird eine optimale Geschmacksmaskierung in der Mundpassage und eine schnelle Freisetzung im Magen erreicht.

Durch den Zusatz einer physiologisch verträglichen Säure oder Base kann durch die Bildung einer schwerer löslichen Form des Wirkstoffes bei Zutritt von Wasser die Geschmacksmaskierung verstärkt werden. Die schwerer lösliche Form kann dabei die freie Wirkstoffbase oder -säure sein oder aber ein schwerer lösliches Salz davon.

Die Herstellung der verdichteten Formlinge aus pulvrigen Substanzen erfolgt mittels Granulation vorzugsweise im Highshear Mixer, Rotorgranulation oder Extrusion. Zur Ausrundung und Glättung der Oberfläche können die Formlinge im Spheronizer ausgerundet werden. Für die spätere Verpressung der gecoateten Formlinge ist eine hohe Dichte und eine möglichst runde Form von entscheidender Bedeutung, denn Formlinge mit hoher Porosität und ungleichmäßiger Form werden so stark verformt, daß der Filmüberzug in Mitleidenschaft gezogen wird.

Die Pellets setzen sich aus 30 bis 98 %, vorzugsweise 50 bis 98 % Wirkstoff, aus 2 bis 70 %, vorzugsweise 2 bis 30 % Bindemittel, aus 0 bis 5 %, vorzugsweise 0,1 bis 1 % Emulgator und gegebenenfalls aus 2 bis 30 %, vorzugsweise 2 bis 15 % Sprengmittel, zusammen sowie gegebenenfalls 0 bis 30 %, vorzugsweise 0 bis 20 % einer physiologisch verträglichen Säure oder Base. Die Angaben beziehen sich auf Gew.-%-Angaben.

Als Wirkstoffe können Nahrungsergänzungs- oder Zusatzstoffe, Vitamine, Mineralstoffe oder Spurenelemente, insbesondere bevorzugt aber pharmazeutische Wirkstoffe, eingesetzt werden.

Unter den pharmazeutischen Wirkstoffen, die eine Geschmacksmaskierung erforderlich machen, versteht man beispielsweise Acetaminophen, Ibuprofen, Naproxen, Chlorpheniramin, Dextromethorphan, Acetylsalicylsäure, Loperamid, Pseudoephedrin, Diphenhydramin, Famotidin, Cimetidin, Ranitidin, Nizatidin, Salze oder auch Gemische davon.

Insbesondere bevorzugt ist die Verwendung des erfindungsgemäßen Polymers zur Geschmacksmaskierung von Ibuprofen und Acetaminophen.

Bindemittel können sein Polyvinylpyrrolidon, Hydroxypropylcellulose, Hydroxypropylmethylcellulose oder Maltodextrin. Als Emulgatoren können Polyethoxylate einer Fettsäure, eines pflanzlichen Öles, eines Fettalkoholes oder einer Sorbitanfettsäureverbindung eingesetzt werden. Salze von Alkylsulfonaten, wie z.B. Natriumlaurylsulfat sind ebenfalls geeignet. Sprengmittel können sein, quervernetztes Polyvinylpyrrolidon, Croscarmellose oder quervernetzte Natriumcarboxymethylstärke.

Zur Herstellung der gecoateten Partikel im Wirbelbett können sowohl das Top spray- als auch das Bottom spray- (Wurster) Verfahren oder Verfahren mit rotierendem Fließbett angewendet werden. Diese Verfahren werden sowohl in "Überzogene Darreichungsformen" herausgegeben von der wissenschaftlichen Verlagsgesellschaft Stuttgart als auch in "Aqueous Polymeric Coatings for Pharmaceutical Dosage Forms" veröffentlicht von Marcel Dekker, Inc.: Coated Pharmaceutical Dosage Forms, CRC Press, Medpharm Scientific Publishers Stuttgart 1998, Pharmaceutical Coating Technology ed. by G. Cole, Taylor and Francis Ltd. 1995, Aqueous Polymeric Coatings for Pharmaceutical Dosage Forms, Marcel Dekker 1997, beschrieben. Die Sprühsuspension wird auf das vorgewärmte, wirbelnde Gut kontinuierlich aufgesprüht. Des weiteren ist ein Coating der Partikel sowohl im Hüttlin Kugelcoater als auch im Rotogranulator möglich.

Zur Erreichung einer Geschmacksmaskierung ist abhängig von der Partikelgröße und -form eine Auftragsmenge von 1 bis 25 Gew.-% notwendig. Die Größe der gewünschten Tablettenform spielt ebenfalls eine Rolle. Kleinere Tabletten werden ohne Zerkauen geschluckt und benötigen somit eine dünnere Coatingschicht als hochdosierte Tabletten, die durch Zerkauen zerkleinert werden und somit länger im Mund verweilen. Die exakte Auftragsmenge muss für jeden Wirkstoff experimentell ermittelt werden.

Für Kautabletten sollten Formlinge mit einer mittleren Teilchengröße kleiner 1,0 mm vorzugsweise kleiner 0,5 mm eingesetzt werden, da hierbei die Gefahr eines Zerkauens geringer ist.

Die Tablettierung der überzogenen Formlinge zu Kautabletten erfolgt mit den üblichen Direkttablettierhilfsstoffen wie z.B. Ludipress, Ludipress LCE, Sorbitol, Mannitol, Dextrose, Saccharose, Isomalt, mikrokristalline Cellulose. Wie üblich können auch Trockenbindemittel, Fließregulierungsmittel, Sprengmittel und Gleitmittel eingesetzt werden.

Durch die Verwendung einer physiologischen Säure oder Base in der Tablettiermischung kann die Geschmacksmaskierung weiter verstärkt werden. Durch diese Zusätze wird beim Zerkauen der Tablette der pH-Wert des Speichels so eingestellt, daß eine niedrige Löslichkeit des Wirkstoffes im Speichel resultiert. Es gelangt dadurch noch weniger Wirkstoff an die Schleimhaut, z.B. kann durch Verwendung von Citronensäure oder Weinsäure die Löslichkeit von Ibuprofen im Speichel erheblich reduziert werden.

Der für die Geschmacksmaskierung entwickelte Filmüberzug ist ebenfalls anwendbar, um z.B. zwei Wirkstoffe in einer Darreichungsform zu isolieren, die Unverträglichkeiten aufweisen.

In besonderen Fällen kann ein zweischichtiger Überzug sinnvoll sein. Dazu wird direkt auf den Kern eine erste Schicht mit einem höheren Anteil an hydrophilem Zusatz gesprüht und anschließend eine zweite mit reduzierten Anteil an hydrophilem Zusatz, im Extremfall sogar ohne hydrophilen Zusatz. Dadurch kann die gesamte Coatingmenge unter Beibehaltung der Geschmacksmaskierung verringert werden:

In den nachfolgenden Beispielen wird die Herstellung und Verwendung des erfindungsgemäßen Coatings näher erläutert.

### Beispiele

Die nachfolgend aufgeführten Beispiele sind auf den Labormaßstab bezogen. Die angewendeten Coatingverfahren entsprechen dem Stand der Technik und werden in Fachbüchern wie z.B. "Überzogene Darreichungsformen" herausgegeben von der wissenschaftlichen Verlagsgesellschaft Stuttgart als auch in "Aqueous Polymeric Coatings for Pharmaceutical Dosage Forms" veröffentlicht von Marcel Dekker, Inc. beschrieben. Das angegebene Verhältnis der eingesetzten Komponenten a : b : c bezieht sich auf Feststoffe.

Die eingesetzten Wirk- und Hilfsstoffe werden anschließend beschrieben:
- Acetaminophen-Granulat, Fa. Knoll AG, Ludwigshafen, Deutschland
- Acetaminophen-Kristalle, Fa. Knoll AG, Ludwigshafen, Deutschland
- Ibuprofen 25, Fa. Knoll AG, Ludwigshafen, Deutschland
- Acetylsalicylsäure-Kristalle, Fa.
- Kollicoat SR 30 D, BASF AG, Ludwigshafen, Deutschland
- Kollidon SR, BASF AG, Ludwigshafen, Deutschland
- Kollidon 30, BASF AG, Ludwigshafen, Deutschland
- Kollidon 90F, BASF AG, Ludwigshafen, Deutschland
- Cremophor RH 40, BASF AG, Ludwigshafen, Deutschland
- Propylenglykol, BASF AG, Ludwigshafen, Deutschland
- Avicel PH 105, Lehmann & Voss, Hamburg, Deutschland
- Pharmacoat 603, Shin-Etsu, Tokyo, Japan
- Aerosil, Degussa-Hüls AG, Frankfurt am Main, Deutschland

### Herstellung gecoateter Formlinge mittels wässriger Sprühsuspension

Die festen Bestandteile der Sprühformulierung werden in Wasser gelöst. Der Weichmacher wird in die Polymerlösung eingebracht und suspendiert. Diese Weichmacherlösung wird unter Rühren in die wässrige Polyvinylacetatdispersion eingetragen. Die Sprühsuspension ist ohne weitere Standzeiten sofort einsetzbar. Die Herstellung der gecoateten Partikel erfolgt zum einen in der Wirbelschicht, wobei sowohl das Top spray- als auch das Bottom spray- (Wurster) Verfahren angewendet werden können als auch in Geräten mit rotierendem Fließbett wie dem Hüttlin Kugelcoater oder dem CF-Coater.

Die Sprühsuspension wird auf das vorgewärmte, wirbelnde Gut kontinuierlich aufgesprüht. Die Einstellung der Parameter ist den jeweiligen Beispieltabellen zu entnehmen. Ein Curing der gecoateten Partikel ist üblicherweise nicht notwendig, kann aber in besonderen Fällen die Geschmacksmaskierung verbessern.

### Beispiel 1

| Zusammensetzung | | | | Verfahrensparameter | | | |
|---|---|---|---|---|---|---|---|
| | [%] | [g] | | Gerät | Aeromatic, Strea 1, Top spray | | |
| Kollicoat | 41,65 | 109,34 | | Einwaage Partikel | 350 | Zulufttemperatur | 60 |
| SR 30 D | | | | [g] | | [°C] | |
| Propylenglykol | 1,25 | 3,28 | | Acetaminophen-Granulat | | Ablufttemperatur | 35-40 |
| | | | | | | [°C] | |
| Kollidon 30 | 6,25 | 16,41 | | Verhältnis a:b:c | 1:0,5:0,1 | Sprühdruck [bar] | 1,2 |
| VE-Wasser | 50,85 | 133,47 | | Auftragsmenge [%] | 15 | Sprührate [g/min] | 3-5 |
| Summe | 100,0 | 262,5 | | Feststoffgehalt d. | 20 | Nachtrocknung | 45°C, 3 |
| | | | | Sprühansatzes [%] | | [°C, min] | |

### Beispiel 2

| Zusammensetzung | | | | Verfahrensparameter | | | |
|---|---|---|---|---|---|---|---|
| | [%] | [g] | | Gerät | Aeromatic, Strea 1, Top spray | | |
| Kollicoat SR | 49,38 | 129,63 | | Einwaage Partikel | 350 | Zulufttemperatur | 60 |
| 30 D | | | | [g] | | [°C] | |
| Propylenglykol | 1,48 | 3,89 | | Acetaminophen-Granulat | | Ablufttemperatur | 35-40 |
| | | | | | | [°C] | |
| Mowiol 4/88 | 3,70 | 9,72 | | Verhältnis a:b:c | 1:0,25:0,1 | Sprühdruck [bar] | 1,2 |
| VE-Wasser | 45,44 | 119,26 | | Auftragsmenge [%] | 15 | Sprührate [g/min] | 3-5 |
| Summe | 100,0 | 262,5 | | Feststoffgehalt d. | 20 | Nachtrocknung | 45°C, 3 |
| | | | | Sprühansatzes [%] | | [°C, mm] | |

Mowiol 4/88 wird unter Erhitzen gelöst. Die weitere Verarbeitung nach Abkühlung des Produkts entspricht der obigen Beschreibung.

### Beispiel 3

| Zusammensetzung | | | | Verfahrensparameter | | | |
|---|---|---|---|---|---|---|---|
| | [%] | [g] | | Gerät | Aeromatic, Strea 1, Top spray | | |
| Kollicoat SR | 51,28 | 134,62 | | Einwaage Partikel | 350 | Zulufttemperatur | 60 |
| 30 D | | | | [g] | | [°C] | |
| Propylenglykol | 1,54 | 4,04 | | Acetaminophen-Granulat | | Ablufttemperatur | 35-40 |
| | | | | | | [°C] | |
| Aerosil | 3,08 | 8,08 | | Verhältnis a:b:c | 1:0,2:0,1 | Sprühdruck [bar] | 1,2 |
| VE-Wasser | 44,10 | 115,76 | | Auftragsmenge [%] | 15 | Sprührate [g/min] | 3-5 |
| Summe | 100,0 | 262,5 | | Feststoffgehalt d. | 20 | Nachtrocknung | 45°C, 3 |
| | | | | Sprühansatzes [%] | | [°C, min] | |

### Beispiel 4

| Zusammensetzung | | | | Verfahrensparameter | | | |
|---|---|---|---|---|---|---|---|
| | [%] | [g] | | Gerät | Aeromatic, Strea 1, Top spray | | |
| Kollicoat SR | 49,38 | 129,63 | | Einwaage Partikel | 350 | Zulufttemperatur | 60 |
| 30 D | | | | [g] | | [°C] | |
| Propylenglykol | 1,48 | 3,89 | | Acetaminophen-Granulat | | Ablufttemperatur | 35-40 |
| | | | | | | [°C] | |
| Avicel PH 105 | 3,70 | 9,72 | | Verhältnis a:b:c | 1:0,25:0,1 | Sprühdruck [bar] | 1,2 |
| VE-Wasser | 45,44 | 119,26 | | Auftragsmenge [%] | 15 | Sprührate [g/min] | 3-5 |
| Summe | 100,0 | 262,5 | | Feststoffgehalt d. | 20 | Nachtrocknung | 45°C, 3 |
| | | | | Sprühansatzes [%] | | [°C, min] | |

### Beispiel 5

| Zusammensetzung | | | | Verfahrensparameter | | | |
|---|---|---|---|---|---|---|---|
| | [%] | [g] | | Gerät | Aeromatic, Strea 1, Top spray | | |
| Kollicoat SR | 49,38 | 129,63 | | Einwaage Partikel | 350 | Zulufttemperatur | 60 |
| 30 D | | | | [g] | | [°C] | |
| Propylenglykol | 1,48 | 3,89 | | Acetaminophen-Granulat | | Ablufttemperatur | 35-40 |
| | | | | | | [°C] | |
| Kollidon CL-M | 3,70 | 9,72 | | Verhältnis a:b:c | 1:0,25:0,1 | Sprühdruck [bar] | 1,2 |
| VE-Wasser | 45,44 | 119,26 | | Auftragsmenge[%] | 15 | Sprührate [g/min] | 3-5 |
| Summe | 100,0 | 262,5 | | Feststoffgehalt d. | 20 | Nachtrocknung | 45°C, 3 |
| | | | | Sprühansatzes [%] | | [°C, min] | |

### Beispiel 6

| Zusammensetzung | | | Verfahrensparameter | | | |
|---|---|---|---|---|---|---|
| | [%] | [g] | Gerät | Aeromatic, Strea 1, Top spray | | |
| Kollicoat SR | 41,65 | 109,34 | Einwaage Partikel | 350 | Zulufttemperatur | 60 |
| 30 D | | | [g] | | [°C] | |
| Propylenglykol | 1,25 | 3,28 | Acetaminophen-Kristalle | | Ablufttemperatur | 35-40 |
| | | | | | [°C] | |
| Kollidon 90 F | 6,25 | 16,41 | Verhältnis a:b:c | 1:0,5:0,1 | Sprühdruck [bar] | 1,2 |
| VE-Wasser | 50,85 | 133,47 | Auftragsmenge [%] | 15 | Sprührate [g/min] | 3-5 |
| Summe | 100,0 | 262,5 | Feststoffgehalt d. | 20 | Nachtrocknung | 45°C, 3 |
| | | | Sprühansatzes [%] | | [°C, min] | |

### Beispiel 7

| Zusammensetzung | | | | Verfahrensparameter | | | |
|---|---|---|---|---|---|---|---|
| | [%] | [g] | | Gerät | Aeromatic, Strea 1, Bottom spray | | |
| Kollicoat SR | 49,38 | 129,63 | | Einwaage Partikel | 350 | Zulufttemperatur | 60 |
| 30 D | | | | [g] | | [°C] | |
| Propylenglykol | 1,48 | 3,89 | | Ibuprofen-Mikropellets | | Ablufttemperatur | 35-40 |
| | | | | | | [°C] | |
| Kollidon 30 | 3,70 | 9,72 | | Verhältnis a:b:c | 1:0,25:0,1 | Sprühdruck [bar] | 1,2 |
| VE-Wasser | 45,44 | 119,26 | | Auftragsmenge [%] | 15 | Sprührate [g/min] | 3-5 |
| Summe | 100,0 | 262,5 | | Feststoffgehalt d. | 20 | Nachtrocknung | 45°C,3 |
| | | | | Sprühansatzes [%] | | [°C, min] | |

### Beispiel 8

| Zusammensetzung | | | | Verfahrensparameter | | | |
|---|---|---|---|---|---|---|---|
| | [%] | [g] | | Gerät | Aeromatic, Strea 1, Bottom spray | | |
| Kollicoat SR | 47,62 | 125,00 | | Einwaage Partikel | 350 | Zulufttemperatur | 60 |
| 30 D | | | | [g] | | [°C] | |
| Propylenglykol | 1,43 | 3,75 | | Ibuprofen-Mikropellets | | Ablufttemperatur | 35-40 |
| | | | | | | [°C] | |
| Kollidon 30 | 0,71 | 1,86 | | Verhältnis a:b:c | 1:0,25:0,1 | Sprühdruck [bar] | 1,2 |
| Avicel PH 105 | 3,57 | 9,37 | | Auftragsmenge [%] | 1 5 | Sprührate [g/min] | 3-5 |
| VE-Wasser | 46,67 | 122,59 | | Feststoffgehalt d. | 20 | Nachtrocknung | 45°C, 3 |
| | | | | Sprühansatzes [%] | | [°C, min] | |
| Summe | 100,0 | 262,5 | | | | | |

### Beispiel 9

| Zusammensetzung, Coating 1 | | | | Verfahrensparameter | | | |
|---|---|---|---|---|---|---|---|
| | [%] | [g] | | Gerät | Aeromatic, Strea 1, Bottom spray | | |
| Kollicoat SR | 41,67 | 109,38 | | Einwaage Partikel | 350 | Zulufttemperatur | 60 |
| 30 D | | | | | | [°C] | |
| Propylenglykol | 1,25 | 3,28 | | Ibuprofen-Mikropellets | | Ablufttemperatur | 35-40 |
| | | | | | | [°C] | |
| Kollidon 30 | 6,25 | 16,41 | | Verhältnis a:b:c | 1:0,25:0,1 | Sprühdruck [bar] | 1,2 |
| VE-Wasser | 50,83 | 133,43 | | Auftragsmenge [%] | 15 | Sprührate [g/min] | 3-5 |
| Summe | 100,0 | 262,5 | | Feststoffgehalt d. | 20 | Nachtrocknung | 45°C, 3 |
| | | | | Sprühansatzes [%] | | [°C, min] | |

| Zusammensetzung, Coating 2 | | | | Verfahrensparameter | | | |
|---|---|---|---|---|---|---|---|
| | [%] | [g] | | Gerät | Aeromatic, Strea 1, Bottom spray | | |
| Kollicoat SR | 49,38 | 17,28 | | Einwaage Partikel | 350 | Zulufttemperatur | 60 |
| 30 D | | | | [g] | | [°C] | |
| Propylenglykol | 1,48 | 0,52 | | Ibuprofen-Mikropellets | | Ablufttemperatur | 35-40 |
| | | | | | | [°C] | |
| Kollidon 30 | 3,70 | 1,30 | | Verhältnis a:b:c | 1:0,25:0,1 | Sprühdruck [bar] | 1,2 |
| VE-Wasser | 45,44 | 15,9 | | Auftragsmenge [%] | 2 | Sprührate [g/min] | 3-5 |
| Summe | 100,0 | 35,0 | | Feststoffgehalt d. | 20 | Nachtrocknung | 45°C, 3 |
| | | | | Sprühansatzes [%] | | [°C, min] | |

Bei allen Beispielen war kein bitterer oder schlechter Geschmack des Arzneistoffes festzustellen.

### Herstellung gecoateter Formlinge mittels organischer Sprühsuspension

Die festen Bestandteile der Sprühformulierung werden in Aceton bzw. Methanol gelöst. Der Weichmacher wird in die Polymerlösung eingebracht und suspendiert. Die Sprühsuspension ist ohne weitere Standzeiten sofort einsetzbar. Die Herstellung der gecoateten Partikel erfolgt zum einen in der Wirbelschicht, wobei sowohl das Top spray- als auch das Bottom spray- (Wurster) Verfahren angewendet werden können als auch im Hüttlin Kugelcoater.

Die Sprühsuspension wird auf das vorgewärmte, wirbelnde Gut kontinuierlich aufgesprüht. Die Einstellung der Parameter ist den jeweiligen Beispieltabellen zu entnehmen. Ein Curing der gecoateten Partikel ist nicht notwendig, eine Nachtrocknung ist empfehlenswert um Restbestandteile des organischen Lösungsmittels restlos zu entfernen.

### Beispiel 10

| Zusammensetzung | | | Verfahrensparameter | | | |
|---|---|---|---|---|---|---|
| | [%] | [g] | Gerät | Aeromatic, Strea 1, Bottom spray | | |
| Kollidon SR | 9,35 | 32,81 | Einwaage Partikel | 350 | Zulufttemperatur | 60 |
| | | | [g] | | [°C] | |
| Propylenglykol | 0,95 | 3,28 | Ibuprofen-Mikropellets | | Ablufttemperatur | 35-40 |
| | | | | | [°C] | |
| Kollidon 30 | 4,70 | 16,41 | Verhältnis a:b:c | 1:0,5:0,1 | Sprühdruck [bar] | 1,2 |
| Aceton | 85,0 | 297,5 | Auftragsmenge [%] | 15 | Sprührate [g/min] | 5-7 |
| Summe | 100,0 | 350,0 | Feststoffgehalt d. | 15 | Nachtrocknung | 45°C, 3 |
| | | | Sprühansatzes [%] | | [°C, min] | |

### Beispiel 11

| Zusammensetzung | | | Verfahrensparameter | | | |
|---|---|---|---|---|---|---|
| | [%] | [g] | Gerät | Aeromatic, Strea 1, Top spray | | |
| Kollidon SR | 11,11 | 38,89 | Einwaage Partikel | 350 | Zulufttemperatur | 60 |
| | | | [g] | | [°C] | |
| Propylenglykol | 1,11 | 3,89 | Acetaminophen-Granulat | | Ablufttemperatur | 35-40 |
| | | | | | [°C] | |
| Avicel PH 105 | 2,78 | 9,72 | Verhältnis a:b:c | 1:0,25:0,1 | Sprühdruck [bar] | 1,2 |
| Methanol | 85,0 | 297,5 | Auftragsmenge [%] | 15 | Sprührate [g/min] | 5-7 |
| Summe | 100,0 | 350,0 | Feststoffgehalt d. | 15 | Nachtrocknung | 45°C, 3 |
| | | | Sprühansatzes | | [°C, min] | |

Es konnte kein bitterer oder schlechter Geschmack des Arzneistoffes festgestellt werden.

### Beispiel 12

| Zusammensetzung | | | Verfahrensparameter | | | |
|---|---|---|---|---|---|---|
| | [%] | [g] | Gerät | Aeromatic, Strea 1, Top spray | | |
| Kollicoat SR | 9,35 | 32,8 | Einwaage Partikel | 350 | Zulufttemperatur | 60 |
| 30 D | | | [g] | | [°C] | |
| Propylenglykol | 0,95 | 3,28 | Acetylsalicylsäure-Kristalle | | Ablufttemperatur | 35-40 |
| | | | | | [°C] | |
| Kollidon 30 | 4,70 | 16,41 | Verhältnis a:b:c | 1:0,5:0,1 | Sprühdruck [bar] | 1,2 |
| VE-Wasser | 85,0 | 297,5 | Auftragsmenge [%] | 15 | Sprührate [g/min] | 3-5 |
| Summe | 100,0 | 350,0 | Feststoffgehalt d. | 15 | Nachtrocknung | 45°C, 3 |
| | | | Sprühansatzes [%] | | [°C, min] | |

### Herstellung von Kautabletten

Die abgewogenen Pulverbestandteile werden über ein Sieb mit der Maschenweite 0,8 mm gegeben und anschließend im Turbula Mischer 10min gemischt. Die Tablettierparameter werden so eingestellt, dass eine ausreichende Bruchfestigkeit bei einem Abrieb <1 % erreicht wird.

| Zusammensetzung | | | Verfahrensparameter | |
|---|---|---|---|---|
| | [%] | [g] | Gerät | Tablettenpresse Korsch EKO |
| Granulat gecoatet | 40,0 | 600,0 | Stempelform: | 16mm biplan mit Facette |
| Ludipress LCE | 59,5 | 892,5 | Bruchfestigkeit [N] | 50-80N |
| Magnesiumstearat | 0,5 | 7,5 | | |
| Summe | 100,0 | 1500,0 | | |

Bei der Einnahme der Tabletten war kein bitterer oder schlechter Geschmack festzustellen.

## Patentansprüche

1. Verfahren zur Herstellung einer geschmackmaskierten oralen Darreichungsform durch Verpressung von mit einem Filmüberzug versehenen wirkstoffhaltigen Partikeln mit üblichen Tablettierhilfsstoffen zu Tabletten, **dadurch gekennzeichnet, dass** die wirkstoffhaltigen Partikel mit einem geschmacksmaskierenden Filmüberzug aus
a) Polyvinylacetat
b) einem hydrophilen Zusatz ausgewählt aus der Gruppe der filmbildenden wasserlöslichen Polymeren, und/oder der Gruppe der wasserunlöslichen, aber quellenden Polymeren und/oder der Gruppe der sehr feinteiligen Puderungsmittel, wobei die filmbildenden wasserlöslichen Polymere ausgewählt sind aus der Gruppe der Poly(vinyllactame), Vinylpyrrolidon-Vinylacetat-Copolymere, Polyvinylalkohole oder Cellulosederivate, als wasserunlösliche, aber stark quellende Polymere quervernetzte Poly(vinyllactame), Cellulose bzw. Cellulosederivate oder Stärkederivate und als feinteilige Puderungsmittel hochdisperse Kieselsäuren, feinteilige Stärken, feinteilige Cellulosen oder feinteilige Salze der Phosphorsäure,
mit einem Gewichtsverhältnis von Polyvinylacetat zu hydrophilem Zusatz von 1:0,1 bis 1:0,75 überzogen werden und in Gegenwart von üblichen Tablettierhilfsmitteln zu Tabletten verpresst werden

2. Verfahren nach Anspruch 1, **dadurch gekennzeichnet, dass** der Filmüberzug 0 bis 20 Gew.-% weitere übliche Coatingbestandteile ausgewählt aus der Gruppe der Weichmacher, Antiklebemittel, Farbstoffe, Benetzungsmittel und Entschäumer und 0 bis 30 Gew.-% einer physiologisch verträglichen Säure enthält.

3. Verfahren nach Anspruch 1 oder 2, **dadurch gekennzeichnet, dass** als wirkstoffhaltige Partikel Formlinge aus
a) 30 bis 98 % Wirkstoff
b) 2 bis 70 % Bindemittel
c) 0,1 bis 5,0 % Emulgator sowie gegebenenfalls
d) 2 bis 30 % Sprengmittel
e) und 0 bis 20 % einer physiologisch verträglichen Säure oder Base,
eingesetzt werden.

4. Verfahren nach einem der Ansprüche 1 bis 3, **dadurch gekennzeichnet, dass** die Auftragsmenge des geschmacksmaskierenden Filmüberzugs 1 bis 25 Gew.-% beträgt.

5. Verfahren nach einem der Ansprüche 1 bis 4, **dadurch gekennzeichnet, dass** die orale Darreichungsform als Wirkstoffe Nahrungsergänzungs- oder Zusatzstoffe, Vitamine, Mineralstoffe oder Spurenelemente oder pharmazeutische Wirkstoffe enthält.

6. Verfahren nach einem der Ansprüche 1 bis 5, **dadurch gekennzeichnet, dass** die orale Darreichungsform als Wirkstoffe pharmazeutische Wirkstoffe enthält.

7. Verfahren nach einem der Ansprüche 1 bis 6, **dadurch gekennzeichnet, dass** die orale Darreichungsform als Wirkstoff Acetaminophen, Ibuprofen, Naproxen, Chlorpheniramin, Dextromethorphan, Acetylsalicylsäure, Loperamid, Pseudoephedrin, Diphehydramin, Famotidin, Cimetidin, Ranitidin, Nizatidin, Salze oder Kombinationen davon enthält.

8. Verfahren nach einem der Ansprüche 1 bis 7, **dadurch gekennzeichnet, dass** die Tablettiermischung 0 bis 40 Gew.-% einer physiologisch verträglichen Säure oder Base enthält.

## Claims

1. A process for producing a taste-masked oral dosage form by compressing active ingredient-containing particles provided with a film coating together with conventional tablet excipients to tablets, wherein the active ingredient-containing particles are coated with a taste-masking film coating composed of
a) polyvinyl acetate
b) a hydrophilic additive selected from the group of film-forming water-soluble polymers and/or from the group of water-insoluble but swelling polymers and/or from the group of very fine-particle dusting agents, where the film-forming water-soluble polymers are selected from the group of poly(vinyllactams), vinylpyrrolidone/vinyl acetate copolymers, polyvinyl alcohols or cellulose derivatives, as water-insoluble but highly swelling polymers crosslinked poly(vinyllactams), cellulose or cellulose derivatives or starch derivatives and as fine-particle dusting agents colloidal silicas, fine-particle starches, fine-particle celluloses or fine-particle salts of phosphoric acid,
with a ratio by weight of polyvinyl acetate to hydrophilic additive from 1 : 0.1 to 1 : 0.75, and compressed to tablets in the presence of conventional tabletting aids.

2. The process acording to claim 1, wherein the film coating comprises from 0 to 20% by weight of further conventional coating ingredients selected from the group of plasticizers, non-stick agents, colorants, wetting agents and antifoams and from 0 to 30% by weight of a physiologically tolerated acid.

3. The process according to claim 1 or 2, wherein shaped articles composed of
a) 30 to 98% active ingredient
b) 2 to 70% binder
c) 0.1 to 5.0% emulsifier and, where appropriate,
d) 2 to 30% disintegrant
e) and 0 to 20% of a physiologically tolerated acid or base, are employed as active ingredient-containing particles.

4. The process according to any of claims 1 to 3, wherein the amount of the taste-masking film coating applied is from 1 to 25% by weight.

5. The process according to any of claims 1 to 4, wherein the oral dosage form comprises as active ingredients food supplements or additives, vitamins, minerals or trace elements or active pharmaceutical ingredients.

6. The process as claimed in any of claims 1 to 5, wherein the oral dosage form comprises active pharmaceutical ingredients as active ingredients.

7. The process as claimed in any of claims 1 to 6, wherein the oral dosage form comprises as active ingredient acetaminophen, ibuprofen, naproxen, chlorpheniramine, dextromethorphan, acetylsalicylic acid, loperamide, pseudoephedrine, diphenhydramine, famotidine, cimetidine, ranitidine, nizatidine, salts or combinations thereof.

8. The process as claimed in any of claims 1 to 7, wherein the tablet mixture comprises from 0 to 40% by weight of a physiologically tolerated acid or base.

## Revendications

1. Procédé pour la préparation d'une forme d'administration orale à goût masqué par compression de particules contenant une substance active, munies d'un revêtement en film, avec des adjuvants de pastillage usuels pour donner des comprimés, **caractérisé en ce que** les particules contenant la substance active sont recouvertes avec un revêtement en film masquant le goût constitué de :
a) acétate de polyvinyle
b) un additif hydrophile sélectionné parmi le groupe des polymères solubles dans l'eau filmogènes, et/ou le groupe des polymères insolubles dans l'eau, mais gonflants, et/ou le groupe des agents de poudrage très finement répartis, les polymères filmogènes solubles dans l'eau étant sélectionnés parmi le groupe des poly(vinyllactames), des copolymères de vinylpyrrolidone-acétate de vinyle, des alcools polyvinyliques ou des dérivés cellulosiques, les polymères insolubles dans l'eau, mais fortement gonflants, étant des poly(vinyllactames) à réticulation transversale de la cellulose ou des dérivés cellulosiques ou des dérivés d'amidon et les agents de poudrage finement répartis étant des acides siliciques à dispersion élevée, des amidons finement répartis, des celluloses finement répartis ou des sels finement répartis de l'acide phosphorique,
avec un rapport de poids de l'acétate de polyvinyle sur l'additif hydrophile de 1:0,1 à 1:0,75 et sont compressées en présence d'adjuvants de pastillage usuels pour donner des comprimés.

2. Procédé selon la revendication 1, **caractérisé en ce que** le revêtement en film contient 0 à 20 % en poids d'autres composants d'enrobage usuels sélectionnés parmi le groupe des plastifiants, des agents antiadhésifs, des colorants, des agents mouillants et des agents antimousses et 0 à 30 % en poids d'un acide physiologiquement compatible.

3. Procédé selon la revendication 1 ou 2, **caractérisé en ce que** l'on utilise des corps de moulage, en tant que particules contenant une substance active, constitués de :
a) 30 à 98 % de substance active
b) 2 à 70 % de liant
c) 0,1 à 5,0 % d'émulsifiant ainsi qu'éventuellement
d) 2 à 30 % d'agent effervescent
e) et 0 à 20 % d'un acide ou d'une base physiologiquement compatible.

4. Procédé selon l'une quelconque des revendications 1 à 3, **caractérisé en ce que** la quantité d'application du revêtement en film masquant le goût est de 1 à 25 % en poids.

5. Procédé selon l'une quelconque des revendications 1 à 4, **caractérisé en ce que** la forme d'administration orale contient, en tant que substances actives, des compléments ou des additifs alimentaires, des vitamines, des substances minérales ou des oligoéléments ou des substances actives pharmaceutiques.

6. Procédé selon l'une quelconque des revendications 1 à 5, **caractérisé en ce que** la forme d'administration orale contient, en tant que substances actives, des substances actives pharmaceutiques.

7. Procédé selon l'une quelconque des revendications 1 à 6, **caractérisé en ce que** la forme d'administration orale contient, en tant que substances actives, de l'acétaminophène, de l'ibuprofène, du naproxène, de la chlorphéniramine, du dextrométhorphane, de l'acide acétylsalicylique, du lopéramide, de la pseudoéphédrine, de la diphéhydramine, de la famotidine, de la cimétidine, de la ranitidine, de la nizatidine, des sels ou des combinaisons de ceux-ci.

8. Procédé selon l'une quelconque des revendications 1 à 7, **caractérisé en ce que** le mélange de pastillage contient 0 à 40 % en poids d'un acide ou d'une base physiologiquement compatible.
